# EUROPEAN PATENT SPECIFICATION

(11) **EP 4 445 909 B1**
(45) Date of publication and mention of the grant of the patent: **18.02.2026**
(21) Application number: 24169036.1
(22) Date of filing: 08.04.2024
(51) Int. Cl.: A61K 38/16, A61K 31/663, A61P 15/12, A61P 19/10, A61K 9/00, A61K 31/675, A61K 38/08

(54) **PHARMACEUTICAL FORMULATION CONSISTING OF C-PEPTIDE COMBINED WITH A BISPHOSPHONATE IN A SINGLE FORM AND ITS USE IN THE TREATMENT OF OSTEOSARCOPENIA**
PHARMAZEUTISCHE FORMULIERUNG AUS EINEM C-PEPTID IN KOMBINATION MIT EINEM BISPHOSPHONAT IN EINER EINZELNEN FORM UND IHRE VERWENDUNG BEI DER BEHANDLUNG VON OSTEOSARCOPENIE
FORMULATION PHARMACEUTIQUE COMPRENANT UN PEPTIDE C COMBINÉ AVEC UN BISPHOSPHONATE SOUS UNE FORME UNIQUE ET SON UTILISATION DANS LE TRAITEMENT DE L'OSTÉOSARCOMENIE

(30) Priority: 14.04.2023 IT 202300007269
(43) Date of publication of application: 16.10.2024
(73) Proprietor: Università Degli Studi Magna Graecia di Catanzaro, 88100 Catanzaro (IT)
(72) Inventor: PUJIA, ARTURO, 88060 GASPERINA (CZ) (IT); MONTALCINI, TIZIANA, 88060 GASPERINA (CZ) (IT); MAUROTTI, SAMANTHA, 88050 SELLIA MARINA (CZ) (IT); GAZZARUSO, CARMINE, 27100 PAVIA (PV) (IT); MAZZA, ELISA, 88056 TIRIOLO (CZ) (IT); PUJIA, ROBERTA, 88100 CATANZARO (IT)
(74) Representative: Primiceri, Maria Vittoria

(56) References cited:
- MAUROTTI SAMANTHA ET AL: "Effects of C-Peptide Replacement Therapy on Bone Microarchitecture Parameters in Streptozotocin-Diabetic Rats", CALCIFIED TISSUE INTERNATIONAL, NEW YORK, NY, US, vol. 107, no. 3, 30 June 2020 (2020-06-30), pages 266 - 280, XP037213629, ISSN: 0171-967X, [retrieved on 20200630], DOI: 10.1007/S00223-020-00716-0
- PUJIA ARTURO ET AL: "An update on the potential role of C-peptide in diabetes and osteoporosis", ENDOCRINE, HUMANA PRESS, INC, US, vol. 58, no. 3, 3 April 2017 (2017-04-03), pages 408 - 412, XP036366444, ISSN: 1355-008X, [retrieved on 20170403], DOI: 10.1007/S12020-017-1286-5
- TONK CHRISTIAN HORST ET AL: "Therapeutic Treatments for Osteoporosis-Which Combination of Pills Is the Best among the Bad?", INTERNATIONAL JOURNAL OF MOLECULAR SCIENCES, vol. 23, no. 3, 26 January 2022 (2022-01-26), pages 1393, XP093087766, DOI: 10.3390/ijms23031393

## Description

"Pharmaceutical formulation consisting of C-Peptide combined with a bisphosphonate in a single form and its use in the treatment of osteosarcopenia"

The present invention relates to a pharmaceutical formulation composed of C-Peptide associated with a bisphosphonate in unique form.

The present invention also relates to the use of a pharmaceutical formulation composed of C-Peptide associated with a bisphosphonate in a unique form in the treatment and/or prevention of osteosarcopenia and/or osteoporosis.

In particular, the present invention relates to a formulation for pharmaceutical use for the prevention and treatment of osteosarcopenia.

As is known, osteoporosis is a disease characterized by reduced mineral density and bone microarchitecture that leads to fractures and affects 50% of postmenopausal women and up to 70% of the elderly. In Italy there are about 100,000 admissions per year for femoral fracture with an estimated cost of 1 billion euros. Motor disability affects more than half of patients in the year following the fracture and only 30-40% of these people independently resume daily activities. There are more than 150,000 cases of permanent disability. The total direct costs for the Italian National Health System amount to about 8.5 billion euros. Mortality is also high as a result of the fracture, resulting in 15-25%. Data and estimates of the European situation show that the number of femur fractures expected in the coming years is constantly increasing. Much of the social and health costs resulting from osteoporosis and its complications are not related to drug costs but to the consequences of osteoporosis itself. Prevention plays a fundamental role and today makes use of care strategies that promote the intake of calcium and vitamin D. In addition, state-of-the-art drugs are now available for treatment that can be prescribed by accredited centres and professionals specialised in the sector. Among these, bisphosphonates are the most prescribed for their efficacy and safety.

However, the risk of fractures remains high even in patients adhering to current pharmacological treatments.

Sarcopenia is a syndrome characterized by the progressive and generalized loss of skeletal muscle mass associated with at least one of the following conditions: reduced muscle strength or reduced physical performance. This condition is associated with an increased risk of adverse events such as physical disability, poor quality of life, falls, hospitalizations and death.

Sarcopenia has a prevalence of about 10.2% in the general population with peaks of 30% in adults over 60 years of age up to 50% in the elderly over 80 years of age. It is the elderly who are most at risk, who can lose up to 10% of their muscle mass in just 3 days of inactivity, following, for example, a hospital admission.

The socio-economic costs of sarcopenia are extremely high, hence the importance of preventing and treating this syndrome.

Although the molecular and cellular etiopathogenic mechanisms of osteosarcopenia are not completely known, we know that the loss of bone and muscle mass is related to age, an unbalanced diet, the reduction of protein synthesis, physical inactivity, the slowing of basal metabolism, chronic diseases and the infiltration of fat into muscle and bone tissue. Bones and cone muscles interconnected both chemically and metabolically as well as physically. Furthermore, specific pathophysiological findings, such as fat infiltration and alterations in stem cell differentiation, are common to both diseases, thus suggesting that sarcopenia and osteoporosis are closely linked. In the presence of osteosarcopenia, therapeutic interventions should aim to improve strength and the amount of muscle mass and not just bone density.

Object of the present invention is to provide an optimized pharmaceutical preparation for use in the treatment of osteosarcopenia.

One of the components of the formulation object of the present invention, peptide-C has been tested and is known for the prevention of diabetes complications, as for example reported in patent document EP11784109A. However, it has never entered clinical practice, as it is not currently in the category of drugs for the treatment of diabetes or its complications. There are currently no known applications of C-peptide for the treatment of osteoporosis or sarcopenia, or osteosarcopenia.

C-peptide for many years has been considered inert and simply involved in the correct folding of the insulin molecule. C-peptide is secreted in equimolar concentrations with insulin; however, its half-life is longer and it has serum concentrations five times higher than insulin itself. Therefore, in diabetes mellitus of type 1 (T1DM), residual beta cell function is assessed by basal and/or stimulated secretion of C-Peptide. C-Peptide levels correlate well with both type of diabetes and duration of disease as **C-**Peptide is absent in T1DM. To Date. It is known that C-peptide specifically binds to cell membranes on G-protein associated receptors and mediates Ca +2-dependent pathways, resulting in activation and increased expression of endothelial nitric oxide synthase Na+/K+-ATPase and other transcription factors involved in cellular antiinflammatory, antioxidant and protective mechanisms.

Bisphosphonates, associated in the pharmaceutical formulation object of the present invention, are known only for the therapy of osteoporosis and in the prevention of fractures. Some applications are described for example in patent document US6596710B1 regarding risedronate, US5914099A for alendronate, and finally patent document US5776499A regarding clodronate.

Therefore, pharmaceutical formulations for the prevention and treatment of osteoporosis and reduction of the risk of fractures, obtained by the combination of the C-peptide molecule and bisphosphonates, are not currently known.

Object of the present invention is to provide an optimized pharmaceutical preparation for use in the treatment of osteosarcopenia.

According to the present invention, a pharmaceutical preparation for use in the treatment of osteosarcopenia, as defined in claim 1, is provided.

For a better understanding of the present invention, a preferred embodiment is now described, by way of nonlimiting example, with reference to the accompanying figures, in which:
- figure 1 shows the chemical structure of some bisphosphonates present as a component of the pharmaceutical formulation, according to the invention;
- figure 2 shows the structure of the C-peptide, component of the pharmaceutical formulation, according to the invention;
- figure 3 shows three-dimensional Micro-CT images after administration of C-Peptide in diabetic rats;
- figure 4 shows a graph relating to the experimental results obtained from the co-treatment with C-Peptide and Risedronate and its effect on protein levels of b-Catenin in Saos-2 cells, according to the invention;

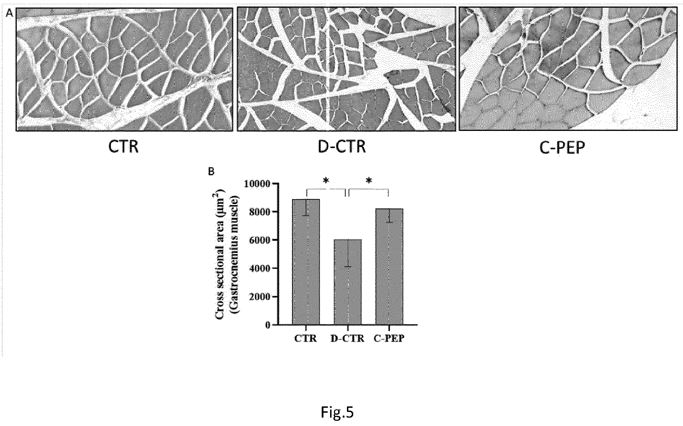

- figure 5 shows experimental results obtained by the Applicant and in particular the effect of the administration of C-peptide on rat gastrocnemius muscle;

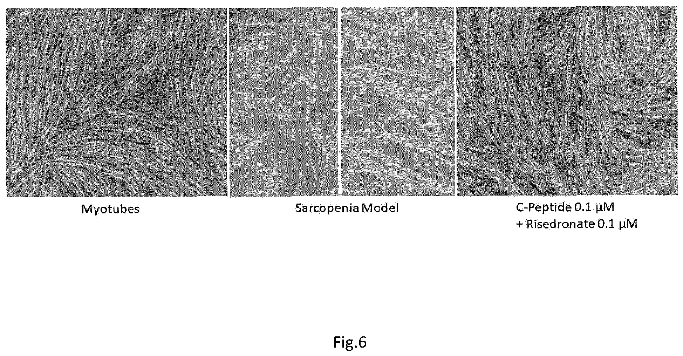

- figure 6 shows further experimental results obtained by the Applicant with the pharmaceutical formulation according to the invention.

The present invention relates to the use of a pharmaceutical formulation comprising C-peptide and at least one bisphosphonate, for the treatment and prevention of osteosarcopenia.

In more detail, the at least one bisphosphonate is selected from the group consisting of:
- Alendonic acid, oral endronate C7H11NO7P2,
- Resendronic acid, or resendronate C4H13NO7P2;
- Clodronic acid, clodronate CH4Cl2O6P2;
- Ibandronic acid, or ibandronate C9H23NO7P2.

From a chemical point of view, bisphosphonates can be considered derivatives of pyrophosphate (chemical structure P2O74-) in which the oxygen that binds both phosphorus atoms has been replaced with a carbon atom, so as to make this type of bond non-hydrolysable.

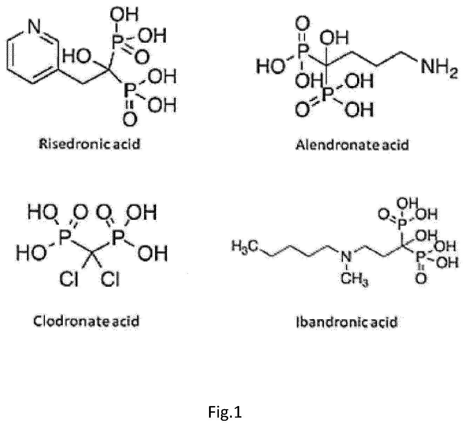

The chemical structures of the most commonly used bisphosphonates are shown in Figure 1. Note that clodronic acid is the only non-aminobisphosphonate.

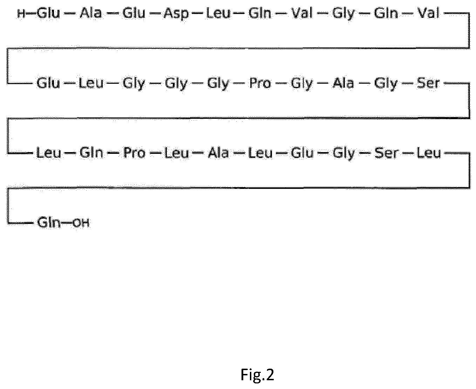

C-Peptide, another component of the pharmaceutical formulation according to the invention, is a 31 amino acid molecule released from pancreatic beta cells during cleavage of the hormone precursor pro-insulin into insulin. Figure 2 shows the structure of the C-peptide.

The synergistic effect of C-peptide and bisphosphonates has been experimentally demonstrated and consists in the fact that the combination between these molecules increases the protein expression of beta-catenin and ERK ½, which represent the main intracellular pathways leading to bone synthesis in osteoblasts and the inhibition of markers of muscle damage, even at lower concentrations of C-peptide and various bisphosphonates.

The pharmaceutical formulation and its specific use, according to the invention, combining C-peptide and bisphosphonates allows to exploit the ability of the latter to bind stably to the bone matrix.

Thus, the pharmaceutical formulation according to the invention allows the C-peptide to be strongly retained in the bone mass and to best exert its effects on the bone-muscle unit.

The pharmaceutical formulation according to the invention consists of C-peptide, a plurality of bisphosphonates, preferably Risedronate, Alendronate, Ibandronate or clodronate, and at least one excipient such as surfactant, aggregant, stabilizer and/or flavoring.

The formulation according to the invention is administrable orally, intramuscularly or parenterally.

The formulation comprises active fragments/peptides of the C-peptide molecule, for example A-F peptides, together with a pharmaceutically acceptable carrier and, optionally, other therapeutic ingredients.

The total amount of active components (C-peptide and one or more Bisphosphonates) in the pharmaceutical formulation is between 99.99% and 0.01% by weight.

When the formulation according to the invention is in a form suitable for parenteral administration, it comprises sterile aqueous solutions and/or suspensions of the pharmaceutically active ingredients, preferably rendered isotonic with the blood of the recipient, generally using sodium chloride, glycerin, glucose, mannitol, sorbitol and/or the like.

The formulation according to one aspect of the invention comprises adjuvants, such as buffers, preservatives, dispersing agents, onset of action or prolonged action promoting agents.

According to one aspect of the invention, the formulation, when suitable for oral administration, comprises active fragments/peptides of the C-peptide molecule in the form of sterile purified stock powder, preferably covered by one or more envelopes that protect from degradation of the active peptides in the stomach and thus allow absorption of these substances from the gum or small intestine.

The synthesis of the C-petpide compound - Risedronate, is obtained, for example, by Fisher esterification.

The compositions according to the present invention are developed by non-recombinant and recombinant methods. Chemical or non-recombinant methods include, but are not limited to, solid-phase SPPs peptide synthesis, solution-phase peptide synthesis, native chemical ligation, gut-mediated protein ligation, or chemical ligation, or a combination thereof. C-peptides are synthesized using standard SPPs, either manually or using commercially available automated SPPs synthesizers.

The Applicant has carried out experimental studies and tests from which it emerges that the use of the pharmaceutical formulation according to the invention in the treatment of osteosarcopenia, allows to prevent the loss of muscle mass of 20-30% and the loss of bone mass of 10% compared to the control group.

Studies with bisphosphonates have shown that a 2-7% change in bone density results in a 30-40% reduction in fracture risk over a period of at least 3 years. Therefore, a remarkable fracture prevention effect linked to the synergistic effect of C-peptide molecules and bisphosphonates is expected.

Experiments in in vitro and in vivo models of osteosarcopenia, and not only osteoporosis or sarcopenia considered in isolation, demonstrate the optimized efficacy of the formulation according to the present invention.

The experimental tests carried out by the Applicant have shown that the administration of 72 nmol/kg/24 h of C-Peptide for 42 days improves the cross-sectional area (CSA), the perimeter and the Minimum Feret diameter of the muscle fibers of the Gastrocnemius (GC), Thiabialis (TA) and the long finger extensor muscle (EDL) in diabetic rats. Specifically, the mean CSA of the GC fibers decreased by 39.5% in the diabetic group, while only 6.6% in the rats of the C-peptide group. The CSA of the TA and EDL muscles was reduced, in the C-peptide group, by 10% and 11%, respectively, while a reduction of 65% and 45% occurred in the diabetic group, compared to the control animals. Similar results were obtained with the other parameters (perimeter and Minimum Feret diameter).

In addition, administration of 72 nmol/kg/24 h of C-peptide for 42 days prevents the increase in serum ubiquitin, induced by diabetes.

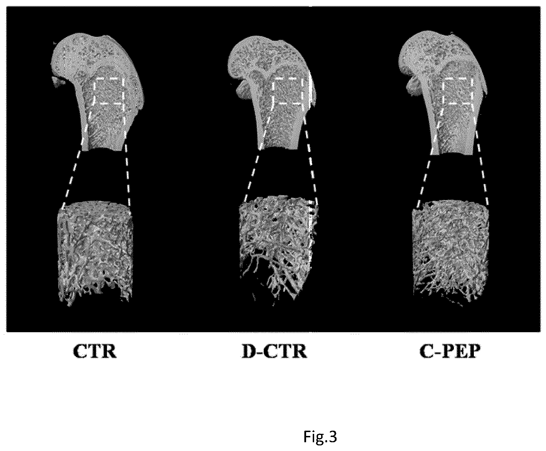

The effect of C-peptide on bone has been experimentally demonstrated. In fact, animals treated with 72 nmol/kg/24 h of C-peptide (C-PEP) showed a better trabecular thickness (p=0.04) and a reduction in trabecular spaces (p=0.01) compared to the diabetes only group (D-CTR), as also shown in the Micro-CT image of Figure 3.

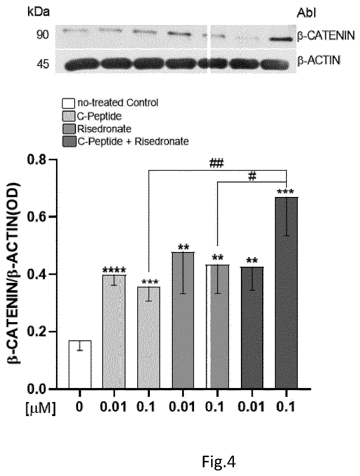

In vitro testing of C-peptide and risedronate co-treatment on bone showed that, on human osteoblast cells (Saos-2), co-treatment with 0.1 µM C-peptide and risedronate statistically significantly increased β-Catenin protein levels (t-student test: * vs. 0; # 0.1 µM C-peptide + risedronate vs. 0.1 µM C-peptide or risedronate). The corresponding experimental results are shown in the graph of Figure 4.

According to one aspect of the invention, the pharmaceutical formulation for oral administration is composed of C-peptide and alendonic acid, or alendronate, for the prevention and treatment of osteoporosis and the reduction of the risk of fractures. The preferred dosage of the formulation for oral administration is in this case 1-5 mg/day of peptide-C and 10 mg/day or 70 mg/1 time per week of alendonic acid.

According to one aspect of the invention, the pharmaceutical formulation for oral administration is composed of C-peptide and resendronic acid, for the prevention and treatment of osteoporosis and the reduction of the risk of fractures. The preferred dosage of the formulation for oral administration is in this case 1-5 mg/day of C-peptide and 5 mg/day or 35 mg/1 time per week or 75 mg/ 1 time per month of resendronic acid.

According to one aspect of the invention, the pharmaceutical formulation for oral administration is composed of C-peptide and clodronic acid, or clodronate, for the prevention and treatment of osteoporosis and the reduction of the risk of fractures. The preferred dosage of the formulation for oral administration is in this case 1-5 mg/day of C-peptide and 200-300 mg/day and of clodronic acid.

According to one aspect of the invention, the pharmaceutical formulation for oral administration is composed of C-peptide and ibandronic acid, or Ibandronate, for the prevention and treatment of osteoporosis and the reduction of the risk of fractures. The preferred dosage compared to ibandronate of the formulation for oral administration is in this case 50 mg/day or 150 mg/1 tablet per month.

According to one aspect of the invention, the pharmaceutical formulation for parenteral administration is composed of C-peptide and clodronic acid, or clodronate, for the prevention and treatment of osteoporosis and the reduction of the risk of fractures. The preferred dosage of the formulation for parenteral administration is in this case 300 mg/day of clodronic acid.

According to one aspect of the invention, the pharmaceutical formulation for intramuscular administration is composed of C-peptide and clodronic acid, or clodronate, for the prevention and treatment of osteoporosis and the reduction of the risk of fractures. The preferred dosage of the formulation for intramuscular administration is in this case 100 mg/day of clodronic acid.

According to one aspect of the invention, the pharmaceutical formulation for the prevention and treatment of osteoporosis and the reduction of the risk of fractures, is composed of C-peptide molecule, also pegylated, and an aminobisphosphonate.

According to one aspect of the invention, the pharmaceutical formulation for the prevention and treatment of osteoporosis and the reduction of the risk of fractures, is composed of a C-peptide molecule, also pegylated, and a bisphosphonate adapted to fix the C-peptide to the bone matrix and muscle fibres.

According to one aspect of the invention, the pharmaceutical formulation is suitable for delayed-release oral or parenteral administration, and comprises C-peptide incorporated in a resorbable matrix, preferably of the polymeric type, which advantageously slows the release of the peptide after administration to the patient.

Thus, the pharmaceutical formulation according to the invention allows to maximize the combined effect of C-peptide and bisphosphonates for the treatment and prevention of osteoporosis and osteosarcopenia.

Finally, it is clear that modifications and variants can be made to the pharmaceutical formulation for the treatment of osteosarcopenia described and illustrated herein without departing from the protective scope of the present invention, as defined in the appended claims.

## Claims

1. Orally, intramuscularly or parenterally administrable pharmaceutical formulation comprising C-peptide and at least one bisphosphonate.

2. Pharmaceutical formulation according to claim 1, for use in the treatment and/or prevention of osteosarcopenia and/or osteoporosis.

3. Pharmaceutical formulation according to claim 1, **characterized in that** the at least one bisphosphonate is selected from the group consisting of:
- Alendonic acid, or alendronate, of the general formula C7H11NO7P2;
- Risendronic acid, or risendronate, of the general formula C4H13NO7P2;
- Clodronic acid, clodronate, of the general formula CH4CI2O6P2;
- Ibandronic acid, or ibandronate, of the general formula C9H23NO7P2.

4. Pharmaceutical formulation according to claim 1, **characterized in that** it consists of Peptide-C, at least one excipient, and a plurality of bisphosphonates, preferably selected from the group consisting of Risedronate, Alendronate, Ibandronate and clodronate.

5. Pharmaceutical formulation according to claim 4, **characterized in that** said at least one excipient is selected from the group consisting of: a surfactant, an aggregator, a stabilizer and/or a flavor.

6. Formulation according to claim 1, **characterized in that** said C-peptide is comprised in the form of active fragments/peptides of the C-peptide molecule and **in that** said formulation further comprises a pharmaceutically acceptable carrier.

7. Formulation according to claim 1, **characterized in that** it comprises a total amount of Peptide-C and of the at least one bisphosphonate comprised between 99.99% and 0.01% by weight.

8. Pharmaceutical formulation according to claim 1, **characterized in that** it comprises at least one additive selected from the group consisting of: adjuvants, buffers, preservatives, dispersing agents, agents promoting the onset of action or prolonged action.

9. Pharmaceutical formulation according to claim 1, **characterized in that** it is suitable for parenteral administration, and comprises sterile aqueous solutions and/or suspensions of Peptide-C and the at least one Bisphosphonate.

10. Pharmaceutical formulation according to claim 1, **characterized in that** it is composed of a C-peptide molecule, also pegylated, and an aminobisphosphonate.

11. Pharmaceutical formulation for use according to claim 2, **characterized in that** it is suitable for oral administration and that said at least one bisphosphonate is alendonic acid, or alendronate, wherein the dosage is between 1 and 5 mg/day of C-peptide and equal to 10 mg/day or 70 mg once per week of alendonic acid.

12. Pharmaceutical formulation for use according to claim 2, **characterized in that** it is suitable for oral administration and that said at least one bisphosphonate is risendronic acid, and wherein the dosage is between 1 and 5 mg/day of C-peptide and equal to 5 mg/day or 35 mg once per week or 75 mg once per month of risendronic acid.

13. Pharmaceutical formulation for use according to claim 2 **characterized in that** it is suitable for oral administration, and **in that** said at least one bisphosphonate is clodronic acid, or clodronate, and wherein the dosage is between 1 and 5 mg/day of C-peptide and between 200 and 300 mg/day of clodronic acid.

14. Pharmaceutical formulation for use according to claim 2, **characterized in that** it is suitable for oral administration, and **in that** said at least one bisphosphonate is ibandronic acid, or ibandronate, and wherein the dosage of ibandronate in the formulation is equal to 50 mg/day or 150 mg/month.

15. Pharmaceutical formulation for use according to claim 2, **characterized in that** it is suitable for parenteral administration and **in that** said at least one bisphosphonate is clodronic acid, or clodronate, and wherein the dosage in clodronic acid is equal to 300 mg/day.

16. Pharmaceutical formulation for use according to claim 2 **characterized in that** it is suitable for intramuscular administration and **in that** said at least one bisphosphonate is clodronic acid, or clodronate, and wherein the dosage of clodronic acid is equal to 100 mg/day.

17. Pharmaceutical formulation according to claim 1, **characterized in that** it is suitable for delayed-release oral or parenteral administration, and that it comprises C-peptide incorporated in a resorbable matrix, preferably of polymeric type.

## Patentansprüche

1. Oral, intramuskulär oder parenteral verabreichbare pharmazeutische Formulierung, umfassend C-Peptid und mindestens ein Bisphosphonat.

2. Pharmazeutische Formulierung nach Anspruch 1 zur Behandlung und/oder Prävention von Osteosarkopenie und/oder Osteoporose.

3. Pharmazeutische Formulierung nach Anspruch 1, **dadurch gekennzeichnet, dass** das mindestens eine Bisphosphonat ausgewählt ist aus der Gruppe bestehend aus:
- Alendonsäure oder Alendronat der allgemeinen Formel C₇H₁₁NO₇P₂;
- Risendronsäure oder Risendronat der allgemeinen Formel C₄H₁₃NO₇P₂;
- Clodronsäure oder Clodronat der allgemeinen Formel CH₄Cl₂O₆P₂;
- Ibandronsäure oder Ibandronat der allgemeinen Formel C₉H₂₃NO₇P₂.

4. Pharmazeutische Formulierung nach Anspruch 1, **dadurch gekennzeichnet, dass** sie aus Peptid-C, mindestens einem Hilfsstoff und mehreren Bisphosphonaten, vorzugsweise ausgewählt aus der Gruppe bestehend aus Risedronat, Alendronat, Ibandronat und Clodronat, besteht.

5. Pharmazeutische Formulierung nach Anspruch 4, **dadurch gekennzeichnet, dass** der mindestens eine Hilfsstoff ausgewählt ist aus der Gruppe bestehend aus: einem Tensid, einem Aggregator, einem Stabilisator und/oder einem Aromastoff.

6. Formulierung nach Anspruch 1, **dadurch gekennzeichnet, dass** das C-Peptid in Form von aktiven Fragmenten/Peptiden des C-Peptid-Moleküls vorliegt und die Formulierung ferner einen pharmazeutisch verträglichen Träger enthält.

7. Formulierung nach Anspruch 1, **dadurch gekennzeichnet, dass** sie eine Gesamtmenge an Peptid-C und des mindestens einen enthaltenen Bisphosphonats zwischen 99,99 Gew.-% und 0,01 Gew.-% enthält.

8. Pharmazeutische Formulierung nach Anspruch 1, **dadurch gekennzeichnet, dass** sie mindestens einen Zusatzstoff enthält, ausgewählt aus der Gruppe bestehend aus: Hilfsstoffen, Puffern, Konservierungsmitteln, Dispergiermitteln, Wirkstoffen zur Förderung des Wirkungseintritts oder der Wirkungsverlängerung.

9. Pharmazeutische Formulierung nach Anspruch 1, **dadurch gekennzeichnet, dass** sie zur parenteralen Verabreichung geeignet ist und sterile wässrige Lösungen und/oder Suspensionen von Peptid-C und mindestens einem Bisphosphonat enthält.

10. Pharmazeutische Formulierung nach Anspruch 1, **dadurch gekennzeichnet, dass** sie aus einem C-Peptid-Molekül, auch pegyliert, und einem Aminobisphosphonat besteht.

11. Pharmazeutische Formulierung zur Verwendung nach Anspruch 2, **dadurch gekennzeichnet, dass** sie zur oralen Verabreichung geeignet ist und dass es sich bei dem mindestens einen Bisphosphonat um Alendonsäure oder Alendronat handelt, wobei die Dosierung zwischen 1 und 5 mg/Tag C-Peptid und 10 mg/Tag oder 70 mg einmal wöchentlich Alendonsäure beträgt.

12. Pharmazeutische Formulierung zur Verwendung nach Anspruch 2, **dadurch gekennzeichnet, dass** sie zur oralen Verabreichung geeignet ist und dass es sich bei dem mindestens einen Bisphosphonat um Risendronsäure handelt, und wobei die Dosierung zwischen 1 und 5 mg/Tag C-Peptid und 5 mg/Tag oder 35 mg einmal wöchentlich oder 75 mg/Monat Risendronsäure beträgt.

13. Pharmazeutische Formulierung zur Verwendung nach Anspruch 2, **dadurch gekennzeichnet, dass** sie zur oralen Verabreichung geeignet ist und dass es sich bei dem mindestens einen Bisphosphonat um Clodronsäure oder Clodronat handelt, wobei die Dosierung zwischen 1 und 5 mg/Tag C-Peptid und zwischen 200 und 300 mg/Tag Clodronsäure beträgt.

14. Pharmazeutische Formulierung zur Anwendung nach Anspruch 2, **dadurch gekennzeichnet, dass** sie zur oralen Verabreichung geeignet ist und dass das mindestens eine Bisphosphonat Ibandronsäure oder Ibandronat ist, und wobei die Dosierung von Ibandronat in der Formulierung 50 mg/Tag oder 150 mg/Monat beträgt.

15. Pharmazeutische Formulierung zur Anwendung nach Anspruch 2, **dadurch gekennzeichnet, dass** sie zur parenteralen Verabreichung geeignet ist und dass das mindestens eine Bisphosphonat Clodronsäure oder Clodronat ist, und wobei die Dosierung von Clodronsäure 300 mg/Tag beträgt.

16. Pharmazeutische Formulierung zur Anwendung nach Anspruch 2, **dadurch gekennzeichnet, dass** sie zur intramuskulären Verabreichung geeignet ist und dass das mindestens eine Bisphosphonat Clodronsäure oder Clodronat ist, und wobei die Dosierung von Clodronsäure 100 mg/Tag beträgt.

17. Pharmazeutische Formulierung nach Anspruch 1, **dadurch gekennzeichnet, dass** sie zur oralen oder parenteralen Verabreichung mit verzögerter Wirkstofffreisetzung geeignet ist und C-Peptid enthält, das in eine resorbierbare Matrix, vorzugsweise polymerer Art, eingebettet ist.

## Revendications

1. Formulation pharmaceutique administrable par voie orale, intramusculaire ou parentérale, comprenant du peptide C et au moins un bisphosphonate.

2. Formulation pharmaceutique selon la revendication 1, destinée au traitement et/ou à la prévention de l'ostéosarcopénie et/ou de l'ostéoporose.

3. Formulation pharmaceutique selon la revendication 1, **caractérisée en ce que** l'au moins un bisphosphonate est sélectionné parmi le group constitué de :
- Acide alendonique, ou alendronate, de la formule générale C₇H₁₁NO₇P₂ ;
- Acide risendronique, ou risendronate, de la formule générale C₄H₁₃NO₇P₂ ;
- Acide clodronique, ou clodronate, de la formule générale CH₄Cl₂O₆P₂ ;
- Acide ibandronique, ou ibandronate, de la formule générale C₉H₂₃NO₇P₂.

4. Formulation pharmaceutique selon la revendication 1, **caractérisée en ce qu'**elle est constituée de peptide C, au moins un excipient, et une pluralité de bisphosphonates, de préférence sélectionnés parmi le risédronate, l'alendronate, l'ibandronate et le clodronate.

5. Formulation pharmaceutique selon la revendication 4, **caractérisée en ce que** ledit au moins un excipient est sélectionné parmi un tensioactif, un agent d'agrégation, un stabilisant et/ou un arôme.

6. Formulation selon la revendication 1, **caractérisée en ce que** ledit peptide C est compris en forme de fragments/peptides actifs de la molécule de peptide C et **en ce que** ladite formulation comprend en outre un excipient pharmaceutiquement acceptable.

7. Formulation selon la revendication 1, **caractérisée en ce qu'**elle comprend une quantité totale de peptide C et d'au moins un bisphosphonate comprise entre 99,99 % et 0,01 % en poids.

8. Formulation pharmaceutique selon la revendication 1, **caractérisée en ce qu'**elle comprend au moins un excipient sélectionné parmi les adjuvants, les tampons, les conservateurs, les agents dispersants, les agents favorisant l'action ou prolongeant l'action.

9. Formulation pharmaceutique selon la revendication 1, **caractérisée en ce qu'**elle est adaptée à l'administration parentérale, et comprend des solutions aqueuses stériles et/ou des suspensions de peptide C et d'au moins un bisphosphonate.

10. Formulation pharmaceutique selon la revendication 1, **caractérisée en ce qu'**elle est constituée d'une molécule de peptide C, également pégylée, et d'un aminobisphosphonate.

11. Formulation pharmaceutique selon la revendication 2, **caractérisée en ce qu'**elle est adaptée à l'administration orale, et que ledit au moins un bisphosphonate est l'acide alendonique ou l'alendronate, dans lesquels la posologie est comprise entre 1 et 5 mg/jour de peptide C et égale à 10 mg/jour ou 70 mg une fois par semaine d'acide alendonique.

12. Formulation pharmaceutique selon la revendication 2, **caractérisée en ce qu'**elle est adaptée à l'administration orale et que ledit au moins un bisphosphonate est de l'acide risendronique, et dans lequel la posologie est comprise entre 1 et 5 mg/jour de peptide C et égale à 5 mg/jour, ou à 35 mg une fois par semaine ou à 75 mg une fois par mois d'acide risendronique.

13. Formulation pharmaceutique selon la revendication 2, **caractérisée en ce qu'**elle est adaptée à l'administration orale, et que ledit au moins un bisphosphonates est de l'acide clodronique ou du clodronate, et dans lesquels la posologie est comprise entre 1 et 5 mg/jour de peptide C et entre 200 et 300 mg/jour d'acide clodronique.

14. Formulation pharmaceutique selon la revendication 2, **caractérisée en ce qu'**elle adaptée à l'administration orale et **en ce que** ledit au moins un bisphosphonate est l'acide ibandronique ou l'ibandronate, et dans lesquels la posologie de l'ibandronate dans la formulation est de 50 mg/jour ou 150 mg/mois.

15. Formulation pharmaceutique selon la revendication 2, **caractérisée en ce qu'**elle est adaptée à l'administration parentérale et **en ce que** ledit au moins un bisphosphonate est l'acide clodronique ou le clodronate, et dans lesquels la posologie de l'acide clodronique est égale à 300 mg/jour.

16. Formulation pharmaceutique selon la revendication 2, **caractérisée en ce qu'**elle adaptée à l'administration intramusculaire et **en ce qu'**au moins un bisphosphonate est l'acide clodronique ou le clodronate, et dans lesquels la posologie de l'acide clodronique est égale à 100 mg/jour.

17. Formulation pharmaceutique selon la revendication 1, **caractérisée en ce qu'**elle est adaptée à une administration orale ou parentérale à libération retardée, et qu'elle comprend du peptide C incorporé dans une matrice résorbable, de préférence de type polymère.
